Europäisches Patentamt

European Patent Office

Office européen des brevets

(19)

(11) Publication number: 0 371 712 A2

## EUROPEAN PATENT APPLICATION

(21) Application number: 89312240.8

(22) Date of filing: 24.11.89

(51) Int. Cl.5: **C12N 15/53, C12N 15/76, C12N 1/21, C12N 9/08, C12P 19/04, C12P 7/24, //(C12N1/21,C12R1:465)**

(30) Priority: 30.11.88 US 277802
16.10.89 US 422023

(43) Date of publication of application:
06.06.90 Bulletin 90/23

(84) Designated Contracting States:
AT BE CH DE ES FR GB GR IT LI LU NL SE

(71) Applicant: IDAHO RESEARCH FOUNDATION, INC.
PO Box 9645
Moscow Idaho 83843(US)

(72) Inventor: Crawford, Donald L.
1988 Appaloosa Road
Moscow Idaho 83843(US)
Inventor: Ramachandra, Muralidhara
1218 South Main No 206
Moscow Idaho 83843(US)

(74) Representative: Harrison, David Christopher et al
MEWBURN ELLIS 2 Cursitor Street
London EC4A 1BQ(GB)

(54) Bacterial extracellular lignin peroxidase.

(57) DNA constructs are provided for the production of Streptomyces lignin peroxidase. The enzyme finds use in the degradation of lignin and oxidation of organic substrates.

EP 0 371 712 A2

## BACTERIAL EXTRACELLULAR LIGNIN PEROXIDASE

The research from which this invention resulted was supported in part by the United States Environmental Protection Agency under Research Contract CR-815300-01-0 and United States Department of Energy Grant DE-FG786ER13586, and National Science Foundation Grant VCS-8807000. The United States Government may have rights in this invention.

The field of the subject invention concerns the expression of lignin peroxidase in a native or heterologous host.

Lignin is a by-product of the production of paper and tends to be an intransigent material with low economic value. Furthermore, there are substantial costs associated with the removal of lignin in the pulping of wood.

Organisms are known which are capable of oxidatively depolymerizing lignin as the organism degrades the cellulose and hemi-cellulose components of plant residues. The depolymerization reactions produce a modified water-soluble acid precipitable polymeric lignin as a major lignin degradation product. Extracellular lignin peroxidases are thought to catalyze the initial catabolism of lignin. The enzymes used by the organism for water-solubilizing lignin could be useful in commercial delignification processes. Therefore, there is substantial interest in the development of enzymatic compositions which may be used in commercial processes for the efficient and economical removal of lignin.

A lignocellulose-induced extra-cellular lignin peroxidase (bacterial lignin peroxidase ALip-P3) in S. viridosporus T7A is reported by Ramachandra et al., Appl. Environ. Microbiol. 1988, 54:3057-3063. See also, Ramachandra et al., Appl. Environ. Microbiol. 1987, 53:2754-2760.

Other lignin-depolymerizing actinomycetes have been described: Thermomonsopora mesophilia, McCarthy et al. 1986, Appl. Environ. Microbiol. Biotechnol., 24:347-352; McCarthy, 1987, FEMS Microbiol. Rev., 46:145-163; Streptomyces badius 252, Phelan et al., Can. J. Microbiol. 1979, 25:1270-1276; Borgmeyer and Crawford, Appl. Environ. Microbiol. 1985, 49:273-278; S. Viridosporus, Adhi et al., 1989, ibid., 55:1165-1168. See also Giroux et al., 1988, ibid, 54:3064-3070 and Ball et al., ibid, 55:1642-1644.

## SUMMARY OF THE INVENTION

DNA sequences and methods employing DNA sequences for identifying lignin peroxidase and expressing lignin peroxidase by recombinant DNA technology is provided. The lignin peroxidase is capable of degrading lignocellulose and the lignin peroxidase gene can be transformed into hosts to enhance lignocellulose degrading activity.

## DESCRIPTION OF SPECIFIC EMBODIMENTS

DNA sequences, DNA constructs, vectors and transformed hosts are provided for the expression of lignin peroxidase, where the enzyme and host capable of expressing the enzyme may be used for the degradation of lignin and lignocellulose.

Lignin peroxidases are found in a number of Streptomyces species including S. viridosporus, S. cyaneus, badius, and other actinomycetes, such as Thermomonosopora mesophilia. The enzymes are characterized by being involved with the initial catabolism of lignin, being lignocellulose-induced, extracellular, catalyzing a carbon-carbon bond cleavage in the side chains in phenolic and non-phenolic lignin substructure models, requiring the presence of hydrogen peroxide or a hydrogen peroxide source and oxidizing polymeric lignin. Induction of production of the subject enzymes may also be induced by lignin, cellulose, larch wood xylan or polymeric lignin degradation intermediates. The peroxidase is a heme protein with broad substrate specificity. Besides lignin and lignin-like products, the subject enzymes will oxidize other phenolics, including chlorinated aromatic compounds. The enzyme has a molecular weight of about 32,000-34,000 daltons, but may be composed of multiple subunits. The enzyme has an activity of greater than 0.30U/min/mg.

The enzyme shows oxidative activity at a pH range of about 4-8, and at a temperature of about 25-50° C. Acidic pn conditions are preferred (optimum pH 5.5-5.6).

The enzyme is obtained from a bacterial source and is capable of degrading both lignin and carbohydrate portions of lignocellulose in the presence of hydrogen peroxide. The enzyme is extracellular, oxidative, inducible by lignin, larch wood xylan, or related substrates and capable of attacking certain lignin substructure chemical bonds that are not degradable by known fungal lignin peroxidases. Isolation, characterization and uses of the enzyme are provided.

The lignin peroxidase enzyme is associated with primary growth of the bacteria. That is, it is presumed to be the result of primary metabolic activity and not dependent upon other factors such as stress to induce production. High concentrations of both organic and inorganic nitrogen in culture media do not inhibit lignin degradation by the enzyme.

The enzymes provided are capable of oxidation and depolymerization of native high molecular weight lignin using hydrogen peroxide as their oxidant. Although other lignin peroxidases have been described, such as in the fungus Phanerochaete chrysosporium, this is the first lignin peroxidase enzyme reported in a bacterium. The bacterial enzyme is capable of attacking certain lignin substructure chemical bonds that fungal lignin peroxidases cannot. It is capable of $C\alpha$-oxidation as well as $C\alpha$-$C\beta$ cleavage of lignin and lignin substructure model compounds.

The instant enzyme is capable of digesting both $\beta$-0-4 and $\beta$-1 type lignin substructure model compounds containing $C\alpha$-hydroxyl or $C\alpha$-carbonyl groups. Oxidative cleavage of $C\alpha$-$C\beta$ bonds of model compounds, 1,2-diarylpropane and arylglcerol-$\beta$-aryl ether types in the presence of hydrogen peroxide is observed. The enzyme preparation catalyzes $C\alpha$-$C\beta$ bond cleavage in the side chains of the diaryl ethers, 1-(3,4-dimethoxyphenyl)-2-(2-methoxyphenoxy)propane-1,3-diol, 1-(4-hydroxy-3-methoxyphenyl)-2-(2-methoxyphenoxy)propan-1-one and a diaryl ethane, 1-(4-methoxyphenyl)-2-(phenyl)ethan-1-one.

This $C\alpha$-$C\beta$ cleavage of $C\alpha$ carbonyl-containing compounds by the bacterial lignin peroxidase enzyme is in direct contrast to the action of lignin peroxidase of P. chrysosporium, which readily cleaved similar compounds only if they contain a hydroxyl group at the $C\alpha$ position. The $C\alpha$- carbonyl group renders aromatic nuclei resistant to oxidation by the fungal lignin peroxidase. Therefore, the bacterial enzyme is more effective at lignin degradation than the fungal counterpart. This degradation of such $C\alpha$-carbonyl-containing compounds during bacterial-mediated lignin degradation is significant because the $C\alpha$-carbonyl content of the lignin increases during degradation by both Streptomyces and Thermomonospora spp.

One characteristic unique to the bacterial lignin peroxidase catalyzed degradation of lignin is the accumulation of acid-precipitable, polyphenolic, polymeric lignin (APPL) intermediates in the growth medium during lignin degradation in addition to the single ring aromatic phenols which are released. These unique degradation intermediates (APPLs) are water-soluable polymers consisting of a heterogenous mixture of molecular weight components of less than about 20KD. The quantity of APPLs is correlated with the biodegradability of the lignocellulose type. Maximal recovery of APPL is obtained from corn lignocellulose, where recovery can reach as high as 30% of the initial lignin present in the substrate. Degraded APPLs are enriched in phenolic hydroxyl groups and, to a small extent, in carboxyl groups. Degradative products are largely oxidative and perhaps involve substantial cleavage of para-hydroxy ether linkages and methoxy groups in lignin.

The instant enzyme is associated with lignin -degrading bacteria. The enzyme is extracellular. When the bacteria is grown in culture, the enzyme occurs in the culture medium.

The lignin peroxidase enzyme is provided in a crude, partially pure, or purified form. Crude extracts of the enzyme may be prepared by growth of suitable bacteria in appropriate growth media. The cultures are grown until maximum levels of peroxidase activity are obtained. The actual time of growth of the cultures to obtain maximum levels of enzyme activity may vary. However, sample results based on shake flask experiments using 2,4-dichlorophenol as substrate show that maximum levels are generally obtained about 48 hrs. to about 96 hrs., usually about 72 hours. Maximum levels of enzyme are generally in the range of about 0.1 U/mg of protein to about 0.18 U/mg of protein, usually, about 0.14 U/mg of protein in medium without lignocellulose. For higher levels of peroxidase activity, the bacteria can be grown in the presence of lignocellulose. Peroxidase activity in the range of about 0.20 U/mg of protein to about 0.32 U/mg of protein, usually about 0.240 U/mg of protein, can be obtained when the bacteria are grown in medium supplemented with about 0.05% (w/vol) lignocellulose.

The addition of larch wood xylan to the bacterial culture in yeast extract-mineral salts medium enhanced extracellular 2,4-DCP peroxidase activity by 3-fold as compared to a medium without xylan, and 2-fold as compared to a medium supplemented with lignocellulose. By using a non-lignin inducer, it is possible to obtain a culture supernatant which does not contain APPL and other water-soluable, partially degraded lignin polymers. The use of a non-lignin inducer is significant as these intermediate polymers interfere with enzyme assays and complicate purification of the peroxidase enzyme.

The crude supernatant, that is, the growth medium containing larchwood xylan of a bacterial culture from which the bacteria and other cell debris have been separated, exhibits a specific activity of about 0.30 U/min/mg. However, the ligninase enzyme may be purified or partially purified by protocols employing conventional purification techniques known in the art, including ammonium sulphate precipitation, sepharose gel permeation chromatography and the like. Purification or partial purification of the lignin peroxidase results in an increase in specific activity of the enzyme. Thus, a specific activity of greater than 0.30 U/min/mg can be obtained upon partial purification. The purified enzyme preparation exhibits a specific activity of at least about 1.02 U/min/mg, resulting in at least a 34-fold increase in specifio activity from the crude supernatant to the purified form of the enzyme.

The specific activities in the partially purified enzyme preparation, as determined by a spectrophotometric assay using 2,4-DCD and L-DOPA as substrate, are presented in Table 1. Additionally, both 2,4-DCP and L-DOPA oxidizing activities are inhibited with known peroxidase inhibitors such as potassium cyanide and sodium azide (Table 1).

The DNA encoding this subject enzyme may be isolated by preparing a library from a lignin peroxidase producing host. The resulting clones are then transformed into a strain of the same or related species that has a negative background for lignin peroxidase activity. The resulting transformants may then be screened using a dye, e.g., blue polymeric Poly B-411, which is a substrate for ligninases. Colonies with clear halos are picked for further investigation. The clones may be further screened with additional dyes, including a variety of phenolic dyes, used by themselves or in conjunction with 4-aminoantipyrine.

In addition, the clones may be further screened to establish the ability to provide lignocellulose degradation. By growing the transformed hosts on a lignocellulose source and comparing the amount of degradation of the lignocellulose as compared to the untransformed parent, the presence of the gene encoding the lignin peroxidase can be established.

The library will usually contain DNA fragments ranging from about 2 to 10 kb, more usually from about `3 to 8 kb. These fragments may be readily excised from the library, once one or more clones have been identified as comprising the lignin peroxidase gene. The fragment may be further reduced in size by employing various nucleases, such as restriction endonucleases, mung bean nuclease, or the like. The resulting fragments may be further screened as described above. Fragments may be tailored by using exonucleases for removing nucleotides at one or both ends of the fragment. Messenger RNA may be obtained from the transformed host and subtracted with cDNA made from the untransformed host in order to enrich for the lignin peroxidase message. The DNA fragment containing the lignin peroxidase may then be hybridized with the mRNA composition having the enriched lignin peroxidase message, so as to define the message, which may then be used to produce cDNA for the gene encoding the lignin peroxidase. In addition, the cDNA for the lignin peroxidase may be used to identify the 5' flanking region which includes the inducible promoter.

The DNA may be used in a variety of constructs. The fragment containing the genomic gene may be used directly by insertion into an appropriate vector for transformation of a competent host. The vector may provide for stable extrachromosomal maintenance or for integration into the genome of the host. One can employ a host which provides the necessary heme prosthetic group to produce the holoenzyme or may provide a host which does not provide the heme prosthetic group resulting in the apoenzyme. As appropriate, the heme prosthetic group may be provided extrinsically, if desired.

The genomic fragment may be further tailored in a variety of ways, as described above, where the 5' flanking region may be removed from the coding region of the gene and replaced with a promoter functional in the transformed host. The promoter may be constitutive or inducible, depending upon the purpose of the transformation. For example, if the organism is to be used for degrading lignocellulose, it may be desirable to have an inducible promoter which responds to the presence of lignocellulose. By contrast, where one wishes only to produce the enzyme and use it extracellularly, a constitutive strong promoter would be desirable. In some instances, it may be desirable to amplify the gene, by integrating the gene into the host genome in conjunction with an amplifying gene, such as DHFR, metallothionein or the like.

A wide variety of expression vectors exist or may be readily prepared for numerous hosts. Vectors are usually characterized by having a replication system which is functional for cloning in a cloning vector, e.g., E. coli, a replication system for the transformed host for expression of the gene, one or more markers for selection in the cloning host and the expression host, one or more polylinkers, particularly where a polylinker is flanked 5' by a promoter region and 3' by a termination region (5'-3' in the direction of transcription).

The markers may provide for stress resistance, such as to antibiotics, heavy metals or other biocide, complementation to an auxotrophic host, virus resistance, or the like.

The various fragments may be joined together by ligation and may be manipulated by primer repair, in vitro mutagenesis, endonuclease restriction, resection, tailing, or the like.

Of particular interest are vectors having a functional replication system capable of stable maintenance in prokaryotes, particularly actinomycetes, more particularly Streptomyces, although other prokaryotes may be of interest, such as E. coli, B. subtilis, B. thermophilous, or even eukaryotic microorganisms, e.g., fungi. Any promoter which is employed will be functional in the expression host. As already indicated, the native promoter may be employed or may be replaced with a different promoter, depending upon the purpose for expression, the particular host, and the like. An illustrative promoter is the $\beta$-galactosidase promoter.

Transformation of the host may be achieved in accordance with conventional ways. With actinomycetes, transformation can be achieved by preparing protoplasts and transforming the protoplasts with a fusogen, e.g., polyethylene glycol, and then regenerating the Streptomyces cells and screening for the presence of the DNA construct. Clones positive for the presence of the DNA construct may then be screened for expression of lignin peroxidase as described above.

Once the host has been transformed, it may be used for the production of the enzyme, which may be isolated in accordance with known ways. Where the enzyme is secreted, it may be isolated from the supernatant by extraction, precipitation, etc., and, if desired, further purified using an affinity column or other technique. Where the enzyme is localized to the cytosol, the cells may be lysed, the lysate freed of the debris and the lignin peroxidase isolated by extraction, protein precipitation, chromatography, or the like.

The transformant or the enzyme may be used for the degradation of lignin or for oxidation of phenolic or other susceptible compounds. The genes may be used to enhance a host already competent in lignin degradation, to impart to a host the partial or complete capability for lignin degradation, where the subject gene may be used by itself or in conjunction with genes for other enzymes involved with lignin degradation, or for the production of the enzyme which may find use in industrial processes as an oxidation catalyst. The enzyme may be obtained in a concentration in the range of about 0.4-0.8 U ml$^{-1}$ (U as defined in the Experimental section) and may be further concentrated and purified.

The following examples are offered by way of illustration and not by way of limitation.

## EXAMPLES

## Example 1

## Enzyme Isolation and Characterization

Culture conditions. S. viridosporus T7A (ATCC 39115) stock cultures were maintained at 4°C on yeast extract-malt extract-glucose agar. Spores from stock slants were inoculated into 2-liter flasks containing 1 liter of 0.6% (wt/vol) yeast extract (Difco Laboratories, Detroit, Mich.) in mineral salt solution plus 0.5% larchwood xylan (Sigma Chemical Co., St. Louis, Mo.) (YMX) and were grown for 3 days at 37°C with shaking at 125rpm.

After 3 days growth, culture supernatant solutions were harvested by filtering through glass wool. Proteins in the filtrate were concentrated 10-fold by ultrafiltration, and were then precipitated with ammonium sulfate (70% saturation). The precipitate was then resuspended in 20mM 2-(N-morpholino)-ethanesulfonic acid (MES) buffer (pH 5.5). Further purification was by gel filtration chromatography, using Sepharose CL-6B (Pharmacia Inc., Piscataway, New Jersey) with MES buffer as eluent. Fractions exhibiting peroxidase activity were pooled and concentrated again by ultrafiltration.

Enzyme assay. Peroxidase activity was routinely assayed with 2,4-dichlorophenol (2,4-DCP) (Sigma) as substrate. A final volume of 1.0 ml of reaction mixture contained 100mM sodium succinate buffer (pH 5.5), 82mM 4-aminoantipyrine (Sigma), 1.0mM 2,4-dichlorophenol, 4.0mM hydrogen peroxide and 100μl of enzyme preparation. The reaction was initiated by the addition of hydrogen peroxide and the increase in $A_{510}$ was monitored for one minute at 37°C. One unit of enzyme was expressed as the amount of enzyme required for an increase of 1.0 absorbance unit per min.

Enzyme activity was also assayed using L-3,4-dihydroxyphenylalanine (L-DOPA; Sigma) as substrate.

Polyacryamide gel electrophoresis (PAGE) and peroxidase staining on gels. Proteins in the enzyme preparation were analyzed by nondenaturing, discontinuous PAGE on a vertical slab gel (7.5% polyacrylamide). After electrophoresis, protein bands were visualized by Coomassie blue staining. Perox-

5

idase bands were developed by activity staining with L-DOPA, 2,4-dichlorophenol, caffeic acid, homoprotocatechuic acid and N,N,N',N'-tetramethylphenylenediamine (TMPD) (all from Sigma) as substrates.

High Pressure Liquid Chromatography (HPLC) of proteins). Proteins in the partially purified and crude enzyme preparations were analyzed on a Hewlett-Packard 1090A high pressure liquid chromatograph equipped with a HP-1040A diode array detector using a Pharmacia FPLC Mono Q anion exchange column. The mobile phase consisted of a gradient of 10mM to 1M sodium acetate, pH 6.0, over a period of 40 min at a flow rate of 1 ml/min. The peaks were monitored at 280 and 409 nm and absorbance spectrum (250-600nm) of each peak was recorded.


## Example 2


Partial purification of peroxidase. 1000 ml of a 3-day YMX-grown culture supernatant fluid was concentrated 10-fold by ultrafiltration, and then subjected to ammonium sulfate precipitation (70% saturation). The precipitate was redissolved in 30 ml of 20mM MES buffer (pH 6.0). This concentrated preparation was applied to a Sepharose CL-6B gel filtration column (1.5 X 100cm). Proteins were eluted from the column with 20mM MES buffer (pH 6.0). The elution profile of the peroxidase from the gel filtration column exhibited the presence of four peroxidase isoforms. Only the major protein peak exhibited 2,4-DCP oxidizing activity. Fractions containing the major peak were pooled and concentrated again by ultrafiltration.

Four peroxidase isoforms from crude, concentrated supernatant fluids were observed when PAGE gels were stained for peroxidase activity with L-DOPA. Among the four isoforms, only the isoform designated P3 was also active against 2,4-DCP, homoprotocatechuic acid, caffeic acid and TMPD. Page gel activity staining with L-DOPA as substrate also showed only P3 in the partially purified preparation, as opposed to all four in the original culture fluid. The P3 band was also the major protein band when the gels were stained with Coomassie blue.

The purification steps described above yielded an enzyme preparation with a 36-fold increase in specific activity. The specific activities in the partially purified enzyme preparation, as determined by spectrophotometric assay using 2,4-DCP and L-DOPA as substrates, are presented in Table 1. Both 2,4-DCP and L-DOPA oxidizing activities were inhibited with known peroxidase inhibitors such as potassium cyanide and sodium azide (Table 1). Similar inhibition of activity was noticed during activity staining of PAGE gels.


## Example 3


### Enzyme Action on Model Compounds


Lignin substructure model compounds. 1-(3,4-Dimethoxyphenyl)-2-(2-methoxyphenoxy)propane-1,3-diol (I) was prepared by sodium borohydride reduction of 1-(3,4-dimethoxyphenyl)-3-hydroxy-2-(2-methoxyphenoxy) propan-1-one, which was synthesized as described by Landucci et al in Holzforschung (1981) 35:67-70. The model compounds 1-(4-methoxyphenyl)-2-(phenyl)ethan-1-one (III) and 1-(4-hydroxy-3-methoxphenyl)-2-(2-methoxyphenoxy)propan-1-one (II) made available by Dr. R. L. Crawford (Department of Bacteriology and Biochemistry, University of Idaho).

Oxidations of lignin model compounds. The model compounds were added to reaction mixtures at a final concentration of 0.02% (wt/vol). Reactions were carried out in a total volume of 3.0 ml, containing 0.1 mM hydrogen pezoxide or a peroxide generating system consisting of 0.02 units/ml of glucose oxidase (Sigma) and 3 mM glucose 0.1 M sodium tartrate buffer (pH 5.5) and 300μl of enzyme preparation. Control reactions were performed with boiled enzyme preparation. Some reaction mixtures were flushed with oxygen and all were incubated at 37°C for 18 hours on a shaker in slanting tubes. The reaction mixtures were then acidified to pH 2-3 with 12 M HCl and extracted once with ether and once with ethyl acetate. Specific products were identified by gas-chromatography/mass-spectrometry (GC/MS) by comparing retention times with those of authentic standards, or by examination of their MS fragmentation patters alone where authentic standards were unavailable, or by HPLC.

Chromatographic analysis. For GC, trimethylsilyl derivatives of extracted samples were prepared with

100 mμl p-dioxane, 10μl pyridine, and 50μl N,O-bis(trimethylsilyl)acetamide.Each sample was held at 35° C for 2 hours prior to injection. GC was performed on a Hewlett-Packard 5890 gas chromatograph with a flame ionization detector and an HP Ultra 2 capillary column (30mm X 0.2mm) (Hewlett Packard Co., Santa Clara, California). Column conditions were as follows. The oven temperature was 120° C for 2 minutes followed by a 15° C per minute gradient to 280° C, which was held for 15 minutes. The injector temperature was 240° C and the detector temperature was 280° C. Mass spectral analysis was performed on a VG-7-7-HG mass spectrometer (VG International, U.K.) at 70 eV, which was coupled to the gas-chromatograph.

HPLC was performed on a Hewlett-Packard 1090A high pressure liquid chromatograph employing a HP-1040A diode array detector. During each run, chromatograms for 258, 280, and 310nm were recorded and the ultraviolet (UV) absorbance spectrum (250-350nm) of each peak was recorded at its front side, apex, and trailing side. A 100mm Hewlett-Packard microbore reverse phase column of Hypersil ODS with 5μM particle diameter was used with a 40° C column temperature, a 5μl sampling loop, and a 0.4 ml/min flow rate. The gradient employed for solvent delivery was a mobile phase consisting of water adjusted to pH 3.2 with $H_2SO_4$ and acetonitrile. The percent of acetonitrile was 10% for 2 minutes, then increased to 5u% over the next 10 minutes, and then increased to 100% over the following 5 minutes. The mobile phase was at 100% acetonitrile for the final 15 minutes; the total run time was 30 minutes. Products were identified by their specific retention times, and comparison of UV spectra with available standard compounds.

Peroxidase reaction with milled corn lignin (MCL) and lignocellulose. The enzyme preparation (300 μ1) was added to a reaction mixture of 3.0 ml containing 10 mg of either MCL (milled corn lignin), extracted corn-stover lignocellulose or cellulose (Whatman cellulose powder, W and R Balston Ltd., U.K.), 0.1 M sodium succinate buffer (pH 5.5), and 1 mM hydrogen peroxide. The reaction mixtures were next incubated at 37° C, while hydrogen peroxide consumption was determined at regular intervals as described by Frew et al., Anal Chim Acta (1983) 155:139-150. At different time intervals, a known volume (100μl) of the reaction mixture was added to 4-aminoantipyrine/phenol reagent and the $A_{505}$ of the resulting solution was measured. Hydrogen peroxide content was calculated from a standard curve.

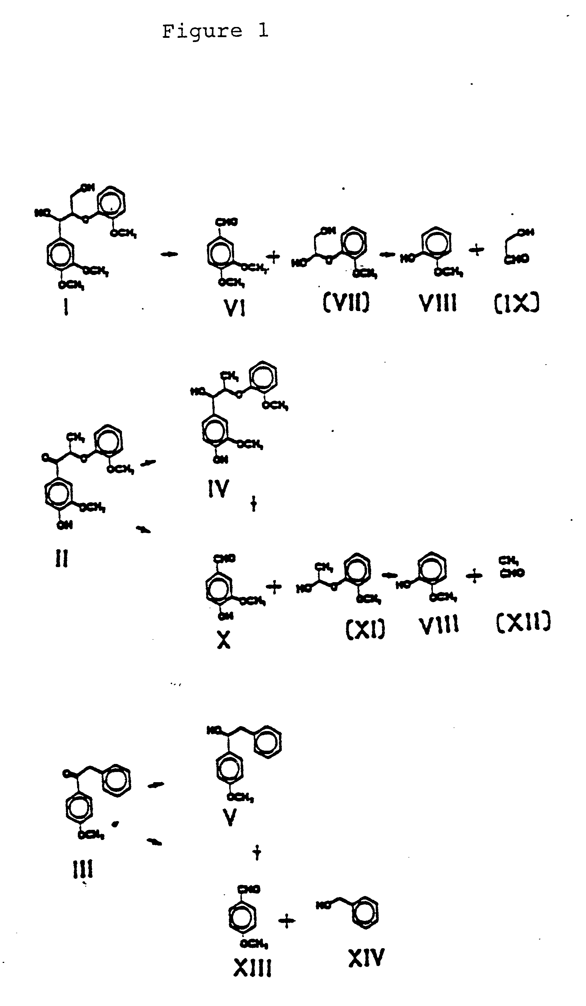

Model compound oxidation. Figure 1 shows the lignin substructure model compounds which were oxidized by the partially purified peroxidase, and products that were formed. Only veratraldehyde, anisaldehyde and vanillin were detected by HPLC, GC, and GC/MS. Guaiacol, formed in trace amounts, was detected by HPLC, GC, and GC/MS. Small amounts of products (IV) and (V) were detected only by MS fragmentation. Minor peaks observed with oxidations of all three model compounds were suspected to be products derived from ring A. None of the above products were detected in the original substrate. The B-aryl ether dimer (I) was cleaved at the $C_\alpha$-$C_\beta$ bond to yield veratraldehyde (VI) and guaiacol (VIII). Vanillin (X) and guaiacol were formed from the diarylether (II), indicating similar $C_\alpha$-$C_\beta$ cleavage. The diarylethane (III) was cleaved between the $C_\alpha$ and $C_\beta$ carbons to yield p-anisaldehyde (XIII) as a major product. Mass spectra of model compound oxidation products are presented in Table 2.

Oxidations of the above lignin substructure model compounds occurred with or without flushing oxygen through the reaction mixture which was incubated on a shaker. Hydrogen peroxide was necessary for the reaction. Addition of hydrogen peroxide twice (once at time zero and once after 9 hr) during the incubation period, or generation of hydrogen peroxide by means of of the glucose-glucose oxidase system, yielded similar results. Enzyme activity was tested with acetate, tartrate, and succinate buffers, at pH 3.5 to 6.5. Among these buffers, sodium tartrate buffer (pH 5.5) was found superior for model compound oxidations, which did not occur above pH 5.5.

Oxidation of lignin. The partially purified enzyme was incubated with extractive-free corn stover lignocellulose, MCL or pure cellulose under the same conditions used for enzyme assays. The rate of hydrogen peroxide consumption during reactions was monitored as an indirect measure of the oxidation of lignin structures (Table 3). Hydrogen peroxide was rapidly utilized when lignin substances (MCL and lignocellulose) were present in the reaction mixture, but not with cellulose powder or in the absence of substrate. Hydrogen peroxide was not consumed in control mixtures lacking enzyme or containing inactivated enzyme (boiled for 10 min).

## Example 4

### Purification of the Lignin Peroxidase

Growth Conditions. Streptomyces viridosporus T7A was grown in a mineral salts-yeast extract (0.6% w/v) medium supplemented with 0.5% (w/v) larch wood xylan. Growth was aerobic with shaking at 37°C. Spores were used as the starting inoculum, and the incubation period was 48 hours.

Quantitative Lignin Peroxidase Assay. The reaction mixture contained 0.16mM 4-aminoantipyrine, 3mM 2,4-dichlorophenol, 5.7 mM $H_2O_2$, and 200$\mu$l of enzyme solution in a final volume of 1.0ml (buffered to pH 6.0). The reaction was initiated by addition of peroxide, and activity was followed by monitoring the change in optical density (O.D.) at 510nm. One unit of activity was defined as the activity resulting in a change in O.D. of 1.0 absorbance unit under these assay conditions.

Lignin Peroxidase Purification and Molecular Weight Determination. After 48 hours of growth, the streptomycete cells were removed from the medium by filtration. The enzyme-containing filtrate was then concentrated by Amicon membrane ultrafiltration using a PM10 Amicon filter. The lignin peroxidase in a 10-fold concentrated filtrate was precipitated by addition of ammonium sulphate to 70% saturation. The precipitate was resuspended in a small volume of 20mM buffer of pH 6.0. Six ml of this enzyme preparation was loaded onto a Sepharose CL-6B 200 gel permeation column (bed volume, 150 cm) and chromatographed at a flow rate of 0.25 ml/min using the pH 6.0 buffer as eluant. Five ml fractions were collected. Lignin peroxidase activity in each fraction was assayed using the following reaction mixture; 2.85mM 2,4-dichlorophenol, 0.23mM 4-aminoantipyrine, 5.7mM $H_2O_2$ in 100 $\mu$l of fraction. The assays were carried out in microtitre plate wells at 37°C using a 5 minute incubation period. Active fractions turned brownish. The most active fraction, as determined by visual examination of the microtitre wells, was retained and assayed quantitatively for activity (see above), protein content (method of Lowry et al. J. Biol. Chem. (1951) 193:265-276), and specific activity (units/min/mg protein). To determine the molecular weight of the lignin peroxidase, molecular weight protein standards (Biorad kit) were subjected to SDS-PAGE gel electrophoresis, and a standard curve was prepared by plotting the log of the known molecular weight of each standard against its distance of migration on the gel. The lignin peroxidase was also subjected to electrophoresis, and its molecular weight was determined from the standard curve. Proteins were generally detected by staining with Coomassie blue. Biorad silver stain was for the determination of protein purity.

We have designated this new lignin oxidizing enzyme as actinomycete lignin peroxidase P3 (ALiP-P3).

The following examples are directed to the genetic engineering in obtaining the gene and demonstrating expression.

## MATERIALS AND METHODS

### Mioroorganisms

Streptomyces viridosporus T7A (ATCC 39115) is a lignin-solubilizing actinomycete (Crawford et al. 1983, Appl. Environ. Microbiol. 45:898-904) originally isolated trom Idaho soil (D.L. Sindin, M.S. thesis, Univ. Idaho 1979). Streptomyces lividans TK64, TK23, and TK 24 were kindly supplied by D.A. Hopwood (John Innes Institute, Norwich, England). Strain TK64 is a proline auxotroph and is resistant to streptomycin (chromosomally encoded), while nonauxotrophic strains TK23 and TK24 express chromosomally encoded resistance to spectinomycin and streptomycin, respectively (Hopwood et al., 1985, Genetic manipulation of Streptomyces: A Laboratory Manual, John Innes Foundation, Norwich, UK). Stock cultures were maintained on yeast extract-malt extract-glucose agar (YEMED) (Ramachandra et al., 1987, supra). Spores from stock slants were used as inoculum in all experiments. Organic medium components were obtained from Difco Laboratories (Detroit, MI).

### Plasmid Vector

The 5.8 kb plasmid pIJ702 (Katz et al. 1983, J. Gen. Microbiol 129:2703-2714) was kindly supplied in Streptomyces lividans TK150 by D.A. Hopwood (John Innes Institute, Norwich, England). The plasmid carries thiostrepton resistance (tsr$^r$) as a selectable marker and tyrosinase (mel$^+$) which is insertionally inactivated during cloning. Stocks of TK150 were maintained on YEMED agar slants containing 50 m$\mu$g ml$^{-1}$ of thiostrepton.

### Plasmid Isolation

For isolation of pJI702, cultures were grown in yeast extract-malt extract-sucrose (YEME) medium with thiostrepton (50 $\mu$g ml$^{-1}$) and 5 mM MgCl$_2$. Plasmid DNA was isolated by the alkaline sodium dodecyl sulfate procedure as described by Hopwood et al. 1985, supra, with some modification (Maniatis et al., 1982, Molecular Cloning: A Laboratory Manual CSHL, Cold Spring Harbor, NY). The full procedure has been previously described (Rafii and Crawford 1988, Appl. Environ. Microbiol. 54:1334-1340).

### DNA Characterizations

Restriction enzyme digestions, agarose gel electrophoresis, DNA labeling with biotin, Southern blot hybridizations, and colony hybridizations were carried out as previously described (Maniatis et al., 1982, supra; Rafii and Crawford 1988, supra.

### Sources of Enzymes, Antibiotic, and Chemicals

Restriction enzymes were purchased from Bethesda Research Laboratories (BRL; Bethesda, MD.), and digestions were carried out under the conditions recommended by the manufacturer. Thiostrepton was obtained from the Squibb Institute for Medical Research (Kalamazoo, MI). Other biochemicals were purchased from commerical sources.

### Cloning and Selection of Transformants

Cloning was carried out as described by Hopwood et al. 1985, supra, and Kendall and Kullum 1984, Gene 29:315-321. Lignin peroxidase negative S. lividans TK64 was used as the recipient, while S. viridosporus T7A was the lignin peroxidase gene donor. The tyrosinase gene of pIJ702 was insertionally inactivated by incorporation of donor DNA fragments into pIJ702 during preparation of theS. viridosporus DNA library. BglII was used to cleave both chromosomal donor DNA (37 ° C; 1 h) and pIJ702 (37 ° C; 2 h)-.The single BglII site in pIJ702 is located within the tyrosinase gene. BglII-digested donor DNA was lofractionated on a sucrose gradient, and 5-10 Kb fragments were ligated into dephosphorylated (alkaline phosphatase for 1 h at 37 ° C) pIJ702 using T4 ligase (16 ° C, overnight). The DNA library in pIJ702 was then transformed into protoplasts of S. lividans TK64 in 15soft agar containing polyethylene glycol (PEG). Transformed protoplasts were regenerated on the R2YE regeneration medium of Hopwood et al., 1985, supra. After 18-22 hours incubation of the R2YE medium at 30 ° C, the agar medium was overlaid with LB soft agar (Hopwood et al., 1985, supra containing thiostrepton (500 $\mu$g/ml).Thiostrepton resistant (tsr$^r$) colonies not expressing tyrosinase (mel$^-$) were considered to be expressing pIJ702 containing donor DNA inserts.

### Screening for Lignin Peroxidase-Expressing Clones

Tsr$^r$/Mel$^-$ colonies from the R2YE medium were transferred to an agar medium containing nitrogen-free mineral salts (Borgmeyer and Crawford 1985, supra), carboxymethyl cellulose (10.0 g L$^{-1}$), yeast extract (10.0 g L$^{-1}$), casamino acids (0.25 g L$^{-1}$), thiostrepton (50 $\mu$g Ml$^{-1}$), and the blue polymeric dye Poly B-411 (0.2 g L$^{-1}$) (Sigma Chem. Co.). The dye is a substrate for ligninases (Sutherland, Abstr. Ann. Meeting Amer. Soc. for Microbiol., 1985, p. 267). Upon oxidation by lignin peroxidase, the dye is decolorized. Transformants producing colonies with clear halos were picked as tentative lignin peroxidase clones.

### Transformation of S. lividans with pIJ702/TK64.1

The transformation-regeneration procedure described above was also used to transform protoplasts of S. lividans strains TK64, TK23, and TK24 with 9.8 kb plasmid pIJ702/TK64.1, which was purified from lignin peroxidase expressing cells of recombinant S. lividans pIJ702/TK64.1 isolated during the present study. Colonies growing on the regeneration medium were transferred to Poly B-411 dye agar plates. After growth for 3-5 days on this medium at 30°C, colonies were examined for clear zones indicative of lignin peroxidase-catalyzed oxidation and decolorization of the Poly B-411.

## Lignin Peroxidase Assay

Lignin peroxidase activity was routinely assayed with 2,4-dichlorophenol (2,4-DCP) (Sigma) as substrate (Ramachandra et al., 1988, supra). A final volume of 1.0 ml of the reaction mixture contained 200 $\mu$l of 100 mM sodium succinate buffer (pH 5.5), 100 $\mu$l of 82 mM 4-aminoantipyrine (Sigma), 100 $\mu$l of 1.0 mM 2,4-DCP, 100 $\mu$l of 4.0 mM hydrogen peroxide, 200 $\mu\mu$l of the enzyme preparation, and 300 $\mu$l of water. The reaction was initiated by addition of hydrogen peroxide, and the increase in $A_{510}$ was monitored for 2 min at 37°C. One unit of enzyme activity was expressed as the amount of enzyme required for an increase in absorbance of 1.0 U min$^{-1}$. Activity could also be measured with L-3,4-dihydroxyphenylalanine (L-DOPA) (Sigma) as substrate (Ramachandra et al., 1987, supra).

## Measurement of Extracellular Peroxide Production

Hydrogen peroxide concentrations in culture filtrates were measured by the method of Frew et al. 1983, Anal. Chem. Acta 155:139-150. At different time intervals, a known volume of filtrate (100 $\mu$l) from cultures of S. viridosporus T7A or S. lividans TK64, grown for 3-7 days in Poly B-411 liquid medium, was added without the dye, to 4-aminoantipyrine-phenol reagent (Frew et al., 1983, supra) also containing horseradish peroxidase (Sigma). The $A_{505}$ of the resulting solution was measured over time at 25°C. Hydrogen peroxide content was correlated with the $A_{505}$ value, or it could be calculated exactly from a standard curve.

## Nondenaturing polyacrylamide Gel Staining Assays

Extracellular proteins were analyzed by nondenaturing, discontinuous polyacrylamide gel electrophoresis (PAGE) on vertical slab gels (7.5% polyacrylamide) (Ramachandra et al., 1987, supra). After electrophoresis, proteins were visualized by silver staining (Hames and Rickwood, 1981, Gel Electroporesis of Proteins: A Practical Approach IRL Press, Oxford). Peroxidase bands on nondenaturing PAGE gels were developed by activity staining as previously described (Ramachandra et al., 1987, supra; Ramachandra et al., 1988, supra.

## Growth of Streptomyces and production of lignin peroxidase using lignocellulose as substrate in solid state culture

S. viridosporus T7A, S. lividans TK64, and S. lividans TK64.1 were grown on 5 gram amounts of ground, extracted corn stover lignocellulose as previously described (CRawford et al., 1983, Appl. Environ. Microbiol. 45:898-904). In one experiment lignin peroxidase was also produced by S. viridosporus T7A in a liquid medium devoid of lignocellulose and consisting of nitrogen-free mineral salts supplemented with yeast extract (Ramachandra et al., 1987, supra).

## RESULTS

Purified lignin peroxidase ALiP-P3 from S. viridosporus T7A in the presence of $H_2O_2$ as cosubstrate, will decolorize the blue dye Poly B-411. The reaction is reversible, and when it is carried out by placing the enzyme and peroxide together in wells cut in the center of Poly B-411 agar plates, an expanding clear halo appears in the blue agar as the enzyme and peroxide diffuse outward. S. viridosporus colonies also decolorize the dye, but the decolorization is more complete and permanent, an indication that enzymes in addition to ALiP-P3 are involved in oxidizing Poly B-411. Colonies of S. lividans TK64 do not decolorize the dye, although the colonies adsorb some dye. No clear halos appear around TK64 colonies during growth on the Poly B-411 medium. Based on these observations, a Poly B-411 decolorization colony screening assay was utilized for selection of lignin peroxidase-expressing clones of S. lividans.

Expression of lignin peroxidase by S. lividans transformants requires the presence of extracellular $H_2O_2$ cosubstrate. S. lividans TK64 produces $H_2O_2$ extracellularly in amounts similar to that excreted by S. viridosporus T7A when growing in the liquid Poly B-411 medium lacking dye or agar. In addition, S. lividans strains produce an extracellular enzyme activity which generates peroxide from various organic substrates. These results indicated that extracellular peroxide concentrations are adequate to supply lignin peroxidase-expressing clones with co-substrate.

Approximately 1000 melanin negative S. lividans TK64 colonies growing on thiostrepton-amended R2YE regeneration medium (presumed to contain S. viridosporus T7A DNA inserts in pIJ702) were picked and transferred to Poly B-411 agar medium plates. Of these clones, two cleared the dye after 3-5 days growth at 30° C. Colonies of these clones produced the same expanding clear halo as observed with pure enzyme. One clone, S. lividans pIJ702/TK64.1, was selected for further characterization, since it grew quickly and sporulated better on agar media than did the other clone.

Tentatively identified lignin peroxidase-expressing recombinant S. lividans TK64.1 was grown in solid state culture on 5 grams of extracted, ground corn stover lignocellulose (Crawford et al. 1983, supra) for 2 weeks at 30° C and then tested for production of extracellular lignin peroxidase. As a positive lignin peroxidase production control, S. viridosporus T7A was grown similarly, but grown at 37° C, while as a negative control, plasmidless S. lividans TK64 was grown similarly. After 2 weeks, cultures were harvested by extraction with 50 ml of 0.1 M phosphate buffer (pH 7.0) and filtration through glass wool. The aqueous filtrates were concentrated 3-fold by dialysis against polyethelene glycol (MW 15,000-20,000) and then assayed for lignin peroxidase activity by the nondenaturing PAGE gel activity staining assay and using the 2,4-DCP oxidation assay. The substrate used to detect peroxidase bands in the gels was L-Dopa (L-3,4-dihydroxyphenylalanine). In the presence of hydrogen peroxide, lignin peroxidase ALip-P3 of S. viridosporus readily oxidizes this substrate, which results in an intense reddish brown color when the reaction is carried out in the presence of 4-aminoantipyrine (Ramachandra et al., 1987, supra).

S. viridosporus filtrates from mineral salts-yeast extract broth-grown cells contained a peroxidase-active band corresponding to the ALip-P3 lignin peroxidase band (Ramachandra et al., 1988 supra). S. viridosporus filtrates from the solid state lignocellulose culture contained significantly greater amounts of the lignin peroxidase and lesser amounts of other L-Dopa-oxidizing peroxidase bands, as has also been observed previously (Ramachandra et al., 1987 supra).

Solid state fermentation filtrates from the plasmidless S. lividans TK64 contained small smounts of several peroxidase active proteins, providing a low background level of peroxidase activity. This activity is not lignin peroxidase, though its presence complicates interpretation of data.

Solid state growth filtrates from S. lividans TK64.1 contained very high levels of peroxidase, and an intensely active band corresponded to the ALip-P3 band observed in S. viridosporus filtrates. Therefore, the PAGE gel assays support the conclusion that the cloned DNA codes for S. viridosporus T7A lignin peroxidase. This conclusion is also supported by the 4-DCP assay results.

Dialysis concentrated filtrates from S. viridosporus T7A, S. lividans TK64, and S. lividans TK64.1 showed 2,4-DCP oxidation activities of 0.10, 0.02, and 0.11 units ml$^{-1}$, respectively. In this experiment, the filtrates were from shake flask cultures grown for 4 days at 30° C (37° C for strain T7A) in the liquid mineral salts-yeast extract (0.6% w/v) supplemented with (0.05% w/v) of lignocellulose. For similar 4 day filtrates from a medium supplemented with carboxymethyl-cellulose (0.05% w/v) instead of lignocellulose, the corresponding values were 0.06, 0.03, and 0.07, respectively.

The plasmid encoding the ALip-P3 gene (pIJ702.LP) was purified from cells of S. lividans and characterized by restriction analysis. The plasmid contained a DNA insert of approximately 4kb and the insert could be excised from pIJ702 with BglII. When the insert was purified, biotinylated, and used to probe S. viridosporus genomic DNA that had been restricted with BglII, the probe hybridized with genomic DNA fragments of approximately 4 kb size. These data confirm that the cloned gene originated from the S. viridosporus genome.

Purified pIJ702.LP was also used to transform S. lividans strains TK64, TK23, and TK24. In these transformations the frequencies of transformation varied, but all transformants expressing pIJ702.LP decolorized Poly B-411 when grown on the dye-containing medium. These results further confirm that the cloned peroxidase gene is responsible for the observed dye decolorization. One TK23 transformant, TK23.1/pIJ702.LP, was retained for further study.

S. lividans TK64 causes little lignocellulose weight loss when grown on corn stover lignocellulose in solid state cultures (S. Eaton, M.S. thesis, Univ. Idaho, 1987). In comparison, S. viridosporus T7A grows vigorously and causes substantial weight loss and loss of both lignin and carbohydrate components of the substrate (Crawford et al., 1983 supra). In a preliminary study, the ability of strain TK23 and recombinant strain TK23.1 to utilize corn stover lignocellulose was compared. Incubations were as solid state cultures.

After 2 weeks of growth at 30°C, the lignocellulose weight loss resulting from growth of the Streptomyces was determined gravimetrically (Crawford et al. 1983 supra). Strains TK23 and TK23.1 generated weight losses of 3.3% and 6.2%, respectively. Uninoculated controls typically show a weight loss of about 3-4%. These data indicate that along with the lignin peroxidase gene of S. viridosporus, the recombinant obtained the ability to at least partially decompose lignocellulose.

It is evident from the above results that the capability of transferring the ability to produce lignin peroxidase has been developed. Thus, by employing the subject constructs, one can greatly enhance the lignin peroxidase capability and production of homologous or heterologous hosts, one can produce the enzyme for treatment of lignin extracellularly, or use the enzyme as a catalytic oxidase.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

Table 1

| Inhibition[a] Of Peroxidase P3 Activities By Cyanide and Azide | | |
|---|---|---|
| | Specific activity (Units/mg protein[b]) | |
| | Substrate | |
| Treatment | 2,4,-DCP | L-DOPA |
| Control (no inhibitor) With potassium cyanide With sodium azide | 1.06 0.45 (58%) 0.18 (83%) | 0.31 0.15 (50%) 0.18 (41%) |

[a]Inhibitors were added at the final concentration of 1mM. Values indicate % inhibition.
[b]Protein content was determined by the Lowry method.

Table 2

| Mass Spectra of Major Products Obtained From Oxidation of Lignin Model Compounds By Peroxidase | |
|---|---|
| Product | Mass spectrum observed m/z (intensity in %) |
| Anisaldehyde (XIII) | 136(M + ,10.0), 135(100), 107(10.3), 92(26.9), 77(34.2 |
| Benzyl alcohol (XIV) (as TMS derivative) | 180(M + ,17.3), 165(35.4), 164(100), 149(16.6), 136(35.3), 135(15.2), 107(45.2), 91(67.3), 82(16.9), 77(17.2), 73(TMS + ,69.1) |
| Guaiacol (VIII) | 196(M + ,0.6), 180(0.3), 165(3.0), 149(1.5), 135(42.8), 91(6.2), 77(20.2), 73(TMS + ,100) |
| Vanillin (X) (as TMS derivative) | 224(M + ,10.1), 223(100), 209(4.4), 179(4.0), 165(8.4), 77(11.6), 73(TMS + ,12.6) |
| Veratraldehyde (VI) | 166(M + ,100), 165(40.4), 151(17.2), 135(22.1), 121(2.5), 95(52.4), 77(29.9) |

Table 3

| Rate of Hydrogen Peroxide Consumption During Peroxidase P3 Reaction With Lignocellulosic Substrates.[a] | | |
|---|---|---|
| | Hydrogen Peroxide Consumption | |
| | (μmol/mg of enzyme/min) | |
| Substrate | With Enzyme | Without Enzyme |
| Lignocellulose | 1.51 | 0.09 |
| MCL | 0.75 | 0.09 |
| Cellulose | 0.76 | 0.09 |
| None | 0.40 | 0.09 |

## Claims

1. A DNA sequence joined to other than a natural flanking sequence encoding an actinomycete lignin peroxidase.

2. A DNA sequence according to Claim 1, wherein said DNA sequence is a sequence of Streptomyces.

3. A DNA sequence according to Claim 2, wherein said Streptomyces is S. viridosporus.

4. A DNA sequence according to Claim 1, wherein said DNA sequence comprises the natural promoter region of said lignin peroxidase.

5. A vector comprising a replication system functional in an actinomycete host and a DNA sequence joined to other than a natural flanking sequence encoding an actinomycete lignin peroxidase.

6. A vector according to Claim 5, wherein said DNA sequence is a sequence of streptomyces.

7. A vector according to Claim 6, wherein said Streptomyces is S. viridosporus.

8. A vector according to Claim 5, wherein said DNA sequence comprises the natural promoter region of said lignin peroxidase.

9. A transformed actinomycete host comprising as a result of transformation a DNA sequence joined to other than a natural flanking sequence encoding an actinomycete lignin peroxidase gene comprising a sequence encoding said lignin peroxidase under the transcriptional and translational control of a promoter and a termination region functional in said host.

10. A transformed actinomycete host according to Claim 9, wherein said DNA sequence comprises a portion of a stable extrachromosomal element.

11. A transformed actinomycete host according to Claim 9, wherein said DNA sequence is a sequence of Streptomyces.

12. A transformed actinomycete host according to Claim 10, wherein said Streptomyces is S. viridosporus.

13. A transformed actinomycete host according to Claim 9, wherein said DNA sequence comprises the natural promoter region of said lignin peroxidase.

14. A transformed prokaryotic host comprising a DNA sequence joined to other than a natural flanking sequence encoding an actinomycete lignin peroxidase gene comprising a sequence encoding said lignin peroxidase under the transcriptional and translational control of a promoter and a termination region functional in said host.

15. A method for producing a lignin peroxidase which comprises:

growing in an appropriate nutrient medium a transformed prokaryotic host according to Claim 14; whereby said lignin peroxidase is produced.

16. A method for producing a lignin peroxidase enzyme having a molecular weight of about 18KD, capable of $C\alpha$-oxidation and $C\alpha$-$C\beta$ cleavage of lignin and lignin degradation products and exhibiting a

EP 0 371 712 A2

specific activity of greater than about 0.30U/min/mg, said method comprising:

growing an Actinomycetales host in an appropriate nutrient medium, whereby said lignin peroxidase is secreted by said host; and

isolating said lignin peroxidase is substantially purified form.

17. A method for oxidizing a phenolic compound compound susceptible to oxidation with a lignin peroxidase in the presence of hydrogen peroxide, said method comprising:

contacting said phenolic compound with a transformed prokaryotic host according to Claim 14 in the presence of a source of hydrogen peroxide;

whereby said phenolic compound is oxidized.

18. A method according to Claim 17, wherein said phenolic compound is lignocellulose.

19. A method for oxidizing a phenolic compound susceptible to oxidation with a lignin peroxidase, said method comprising:

contacting said phenolic compound with a lignin peroxidase produced according to the method of Claim 15 in the presence of a source of hydrogen peroxidase;

whereby said phenolic compound is oxidized.

20. A lignin peroxidase enzyme having a molecular weight of about 18KD, capable of $C\alpha$-oxidation and $C\alpha$-$C\beta$ cleavage of lignin and lignin degradation products, and exhibiting a specific activity of greater than 0.30U/min/mg.

21. The enzyme according to Claim 20, wherein said enzyme is isolated from a member of Actinomycetales.

22. The enzyme according to Claim 21, wherein said member is from Streptomyces.

23. The enzyme, according to Claim 22, wherein said member is from Streptomyces viridosporus

14

# Figure 1